(19) **Europäisches Patentamt / European Patent Office / Office européen des brevets**

(11) **EP 4 317 189 A1**

(12) **EUROPEAN PATENT APPLICATION**
published in accordance with Art. 153(4) EPC

(43) Date of publication:
07.02.2024 Bulletin 2024/06

(21) Application number: 22773394.6

(22) Date of filing: 24.03.2022

(51) International Patent Classification (IPC):
C07K 16/30 (2006.01)　　A61K 39/395 (2006.01)
A61P 1/00 (2006.01)　　A61P 1/02 (2006.01)
A61P 35/00 (2006.01)　　C12N 15/13 (2006.01)
G01N 33/531 (2006.01)　　G01N 33/574 (2006.01)

(52) Cooperative Patent Classification (CPC):
A61K 39/395; A61P 1/00; A61P 1/02; A61P 35/00;
C07K 16/00; C07K 16/30; G01N 33/531;
G01N 33/574

(86) International application number:
PCT/JP2022/013783

(87) International publication number:
WO 2022/202953 (29.09.2022 Gazette 2022/39)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 26.03.2021 JP 2021052723

(71) Applicant: National University Corporation Tokai
National
Higher Education and Research System
Nagoya-shi, Aichi 464-8601 (JP)

(72) Inventors:
• TAKEUCHI, Tamotsu
Gifu-shi, Gifu 501-1193 (JP)
• SAIGO, Chiemi
Gifu-shi, Gifu 501-1193 (JP)
• KITO, Yusuke
Gifu-shi, Gifu 501-1193 (JP)

(74) Representative: Regimbeau
20, rue de Chazelles
75847 Paris Cedex 17 (FR)

Remarks:
The complete document including Reference
Table(s) and the Sequence Listing(s) can be
downloaded from the EPO website

(54) **ANTIBODY OR FRAGMENT THEREOF, AND METHOD FOR USING SAME**

(57)　Provided is an antibody or fragment thereof that recognizes a colorectal cancer-specific or oral cancer-specific antigen. Specifically, provided is an antibody or fragment thereof that recognizes an N-linked glycan that annexin A2 has.

Fig. 7

Colorectal Cancer Tumor Suppression Effect
of Antibody 12G5A

EP 4 317 189 A1

**Description**

Technical Field

The present disclosure relates to an antibody or fragment thereof, and the like. More specifically, the present disclosure relates to an antibody or fragment thereof that recognizes a colorectal cancer- or oral cancer-specific antigen, use of the antibody or fragment thereof, and the like.

Background Art

Antibodies (in particular, monoclonal antibodies) are widely used for early diagnosis of cancer, specific diagnosis, screening, and identification of molecular targets for cancer treatment. In recent year, humanization of monoclonal antibodies has become feasible, and various humanized monoclonal antibodies are used in the treatment of malignant tumors and autoimmune diseases (e.g., CD3 (Orthoclone OKT3), glycoprotein IIb/IIIa receptor (ReoPro), ERBB2 (Herceptin), CD20 (Rituxan), CD25 (Zenapax and Simulect), RSVgpF (Synagis), TNF-$\alpha$ (Remicade), and IL-6R).

For the application of antibodies, it is necessary to establish antibodies that specifically recognize cancer-specific antigens of various tumors, and the specificity of antibodies is an important issue for the clinical application of antibodies. Many studies have been conducted to identify novel molecules specifically expressed on the surface of cancer cells or in the serum of cancer patients; however, most of the studies are not for efficiently identifying molecules or epitopes that are considered to be cancer-specific markers.

Citation List

Patent Literature

PTL 1: WO2015/119180

Summary of Invention

Technical Problem

An object of the present disclosure is to provide an antibody or fragment thereof that recognizes a colorectal cancer- or oral cancer-specific antigen.

Solution to Problem

The present inventors found that antibody 12G5A reacts with human colon adenocarcinoma-derived HT-29 cells, but does not react with differentiated HT-29 cells treated with sodium butyrate (normal intestinal epithelial cells). Based on this finding, the inventors made further improvements.

The present disclosure encompasses, for example, the subject matter described in the following items.

Item 1.

An antibody or fragment thereof that recognizes an N-linked glycan that annexin A2 has.

Item 2.

The antibody or fragment thereof according to Item 1, wherein the antibody or fragment thereof is an antibody or fragment thereof of (I) or (II):

(I) an antibody or fragment thereof comprising a light-chain variable region consisting of the amino acid sequence set forth in SEQ ID NO: 1 and a heavy-chain variable region consisting of the amino acid sequence set forth in SEQ ID NO: 2; or
(II) an antibody or fragment thereof comprising a light-chain variable region consisting of an amino acid sequence having at least 90% identity to the amino acid sequence set forth in SEQ ID NO: 1 and a heavy-chain variable region consisting of an amino acid sequence having at least 90% identity to the amino acid sequence set forth in SEQ ID NO: 2.

Item 3.

The antibody or fragment thereof according to Item 1 or 2, wherein the annexin A2 is a protein comprising the amino acid sequence set forth in SEQ ID NO: 3.

Item 4.

A pharmaceutical composition comprising the antibody or fragment thereof according to any one of Items 1 to 3.

Item 5.

The pharmaceutical composition according to Item 4, which is for use in diagnosis of cancer or for use in treatment of cancer.

Item 6.

The pharmaceutical composition according to Item 5, wherein the cancer is colorectal cancer or oral cancer.

Item 7.

Use of annexin A2 having an N-linked glycan as a colorectal cancer or oral cancer marker.

Item 8.

The use according to Item 7, wherein the annexin A2 is a protein comprising the amino acid sequence set forth in SEQ ID NO: 3.

Advantageous Effects of Invention

Provided is an antibody or fragment thereof that recognizes a colorectal cancer- or oral cancer-specific antigen (specifically, an N-linked glycan that annexin A2 has). Also provided is a pharmaceutical composition comprising the antibody or fragment thereof. Further provided is a method of use of annexin A2 having an N-linked glycan as a colorectal cancer or oral cancer marker.

Brief Description of Drawings

Fig. 1 shows the results of immunofluorescence staining, and the analyzed amino acid sequence (SEQ ID NO: 1) of the light-chain variable region and the analyzed amino acid sequence (SEQ ID NO: 2) of the heavy-chain variable region.
Fig. 2 shows the amino acid sequence of human annexin A2 and the results of Western blotting.
Fig. 3 shows overall survival and progression-free survival (PFS) curves based on 12G5A immunoreactivity in colorectal cancer.
Fig. 4 shows the results of comparing immunoreactivity with 12G5A and immunoreactivity with a commercially available antibody against annexin A2.
Fig. 5 shows the results of comparing 12G5A immunoreactivity in oral non-neoplastic stratified squamous epithelium (A), dysplastic epithelium (B), and oral squamous cell carcinoma (C).
Fig. 6 shows the results of Western blotting and the results of immunofluorescence staining.
Fig. 7 shows the results of measuring the colorectal cancer tumor volume when antibody 12G5A was administered.

Description of Embodiments

Embodiments encompassed by the present disclosure are described in more detail below.
The present disclosure encompasses an antibody or fragment thereof that recognizes an N-linked glycan that annexin A2 has. In the present specification, the antibody or fragment thereof may be referred to as "the antibody of the present disclosure," "the antibody fragment of the present disclosure," "the antibody or fragment thereof of the present disclosure," and the like.

Annexin A2 is a calcium ion ($Ca^{2+}$)- and phospholipid-binding protein with a molecular weight of 36 kDa. Annexin A2 is known to show an increased expression level in cancer cells and is believed to be expressed on the cell membrane.

SEQ ID NO: 3 shows the amino acid sequence of human-derived annexin A2.

The N-linked glycan is attached to an asparagine residue of the amino acid sequence that constitutes annexin A2. In the amino acid sequence that constitutes annexin A2, the asparagine residue to which the glycan is attached may be, for example, the asparagine residue that constitutes the amino acid sequence represented by Asn-X-Ser or Asn-X-Thr (wherein X is any amino acid residue). For example, the N-linked glycan may be attached to the asparagine residue at position 62 of the amino acid sequence set forth in SEQ ID NO: 3.

The N-linked glycan includes N-acetylglucosamine (GlcNAc). Specific examples include a high-mannose type having a structure in which an oligomer of mannose is bound to diacetylchitobiose (GlcNAc-GlcNAc), a complex type having a structure in which mannose and at least one member selected from the group consisting of N-acetylglucosamine (GlcNAc), galactose, and sialic acid are bonded to diacetylchitobiose (GlcNAc-GlcNAc), a hybrid type having a structure in which the high-mannose type and the complex type are mixed with diacetylchitobiose (GlcNAc-GlcNAc), and the like.

The antibody of the present disclosure may be a polyclonal antibody or a monoclonal antibody. Of these, it is preferred that the antibody of the present disclosure is a monoclonal antibody.

Examples of isotypes of the antibody (immunoglobulin) of the present disclosure include IgA, IgD, IgE, IgG (IgG1, IgG2, IgG2a, IgG2b, IgG3, IgG4), IgM, and the like.

Regarding the origin of the antibody of the present disclosure, the antibody of the present disclosure may be, for example, a human-derived antibody, a mouse-derived antibody, a rat-derived antibody, a rabbit-derived antibody, a monkey-derived antibody, a chimpanzee-derived antibody, or the like.

The antibody of the present disclosure may be a chimeric antibody. "Chimeric antibody" refers to an antibody having a constant region that is an amino acid sequence derived from a human and a variable region that is an amino acid sequence derived from a non-human species (e.g., a mouse). The antibody of the present disclosure may also be a humanized antibody.

Examples of the antibody fragment of the present disclosure include Fab, Fab', F(ab')$_2$, Fv, scFv, and the like.

The antibody or fragment thereof of the present disclosure may be (I) an antibody or fragment thereof comprising a light-chain variable region consisting of the amino acid sequence set forth in SEQ ID NO: 1 and a heavy-chain variable region consisting of the amino acid sequence set forth in SEQ ID NO: 2. In the present specification, the antibody or fragment thereof may be referred to as "the antibody or fragment thereof (I) of the present disclosure."

The antibody or fragment thereof of the present disclosure may be (II) an antibody or fragment thereof comprising a light-chain variable region consisting of an amino acid sequence having at least 90% identity to the amino acid sequence set forth in SEQ ID NO: 1 and a heavy-chain variable region consisting of an amino acid sequence having at least 90% identity to the amino acid sequence set forth in SEQ ID NO: 2. In the present specification, the antibody or fragment thereof may be referred to as "the antibody or fragment thereof (II) of the present disclosure."

In the antibody or fragment thereof (II) of the present disclosure, the identity to the amino acid sequence set forth in SEQ ID NO: 1 is, for example, preferably at least 90%, and more preferably at least 95%.

In the antibody or fragment thereof (II) of the present disclosure, the identity to the amino acid sequence set forth in SEQ ID NO: 2 is, for example, preferably at least 90%, and more preferably at least 95%.

The identity of the amino acid sequence can be calculated with default parameters of homology algorithm BLAST (Basic Local Alignment Search Tool) of the National Center for Biotechnology Information (NCBI) in the US (http://www.ncbi.nlm.nih.gov/BLAST/).

The antibody or fragment thereof (II) of the present disclosure may comprise a light-chain variable region consisting of an amino acid sequence in which at least one amino acid is deleted, substituted, or added in the amino acid sequence set forth in SEQ ID NO: 1, and a heavy-chain variable region consisting of an amino acid sequence in which at least one amino acid is deleted, substituted, or added in the amino acid sequence set forth in SEQ ID NO: 2.

In each of the amino acid sequences in which at least one amino acid is deleted, substituted, or added, the upper limit of the number of amino acids deleted, substituted, or added is, for example, 25, and preferably about 10.

More specifically, the number of amino acids deleted, substituted, or added in the amino acid sequence set forth in SEQ ID NO: 1 may be, for example, 1 to 18, 1 to 15, 1 to 10, 1 to 5, or 1 to 3.

More specifically, the number of amino acids deleted, substituted, or added in the amino acid sequence set forth in SEQ ID NO: 2 may be, for example, 1 to 24, 1 to 20, 1 to 18, 1 to 15, 1 to 10, 1 to 5, or 1 to 3.

It is known in this technical field how to make a mutation such as deletion, substitution, or addition of at least one amino acid in a specific amino acid sequence, and any technique can be used. Such a mutation can be made by using, for example, the restriction enzyme treatment; the treatment using an exonuclease, DNA ligase, etc.; site-directed mutagenesis; or random mutagenesis.

When at least one amino acid is substituted, the substitution of the amino acid may be a conservative substitution. In the present specification, "conservative substitution" means the substitution of an amino acid with an amino acid having a side chain with properties similar to those of the side chain of the amino acid. Specific examples of conservative substitutions include substitutions between amino acid residues having a basic side chain, such as lysine, arginine, and histidine; substitutions between amino acid residues having an acidic side chain, such as aspartic acid and glutamic acid; substitutions between amino acid residues having an uncharged polar side chain, such as glycine, asparagine, glutamine, serine, threonine, tyrosine, and cysteine; substitutions between amino acid residues having a nonpolar side chain, such as alanine, valine, leucine, isoleucine, proline, phenylalanine, methionine, and tryptophan; substitutions between amino acid residues having a $\beta$-branched side chain, such as threonine, valine, and isoleucine; substitutions between amino acid residues having an aromatic side chain, such as tyrosine, phenylalanine, tryptophan, and histidine; and the like.

In the amino acid sequence having at least 90% identity to the amino acid sequence set forth in SEQ ID NO: 1 in the antibody or fragment thereof (II) of the present disclosure, for example, the amino acid sequence at position 1 to position 10, the amino acid sequence at position 100 to position 107, or the amino acid sequence at position 125 to position 136 in the amino acid sequence set forth in SEQ ID NO: 1 may be conserved. Any one of the amino acid sequences may be conserved, any two of the amino acid sequences may be conserved, or all three of the amino acid sequences may be conserved. It is preferred that the amino acid sequence at position 100 to position 107 in the amino acid sequence set forth in SEQ ID NO: 1 is conserved.

In the amino acid sequence having at least 90% identity to the amino acid sequence set forth in SEQ ID NO: 2 in the antibody or fragment thereof (II) of the present disclosure, for example, the amino acid sequence at position 1 to position 6, the amino acid sequence at position 47 to position 54, the amino acid sequence at position 71 to position 77, or the amino acid sequence at position 118 to position 144 in the amino acid sequence set forth in SEQ ID NO: 2 may be conserved. Any one of the amino acid sequences may be conserved, any two of the amino acid sequences may be conserved, any three of the amino acid sequences may be conserved, or all four of the amino acid sequences may be conserved. It is preferred that the amino acid sequence at position 47 to position 54 in the amino acid sequence set forth in SEQ ID NO: 2 or the amino acid sequence at position 71 to position 77 in the amino acid sequence set forth in SEQ ID NO: 2 is conserved.

The antibody of the present disclosure can be prepared by a known method. For example, a polyclonal antibody can be prepared by immunizing a mammal with a lysate of cells that express annexin A2 having an N-linked glycan (e.g., colorectal cancer cells) or the like as an immunizing antigen, or, for example, a monoclonal antibody can be prepared by preparing a hybridoma.

The antibody fragment of the present disclosure can be prepared by a known method. For example, the antibody fragment of the present disclosure can be obtained by treating the antibody obtained by any of the methods described above with an enzyme.

The present disclosure also includes a pharmaceutical composition comprising the antibody or fragment thereof. In the present specification, the pharmaceutical composition may be referred to as "the pharmaceutical composition of the present disclosure."

The pharmaceutical composition of the present disclosure may comprise one of the aforementioned antibodies or fragments thereof alone, or may comprise two or more of the aforementioned antibodies or fragments thereof in combination.

The content of the antibody or fragment thereof in the pharmaceutical composition of the present disclosure is not particularly limited and can be appropriately set to not greater than 100 mass%. For example, the content of the antibody or fragment thereof may be about 0.0001 to 99.9 mass%.

The pharmaceutical composition of the present disclosure comprises the antibody or fragment thereof and may further comprise other components. Examples of the other components include pharmaceutically acceptable base substances, carriers, solvents, dispersants, emulsifying agents, buffers, stabilizers, excipients, binders, disintegrators, lubricants, thickeners, antioxidants, preservatives, coating agents, coloring agents, gastric mucosal protective agents, and like drugs. These components may be used singly or in a combination of two or more.

The dosage form of the pharmaceutical composition of the present disclosure is not particularly limited. Examples include tablets, pills, capsules, powders, fine granules, granules, liquids, troches, jellies, injections, plasters, extracts, suppositories, suspensions, tinctures, ointments, poultices, nasal drops, inhalants, liniments, lotions, aerosols, and the like.

The pharmaceutical composition of the present disclosure can be prepared according to a usual method by using the antibody or fragment thereof, if necessary in combination with other components.

As shown in the Examples described later, the antibody or fragment thereof of the present disclosure reacts with cancer cells or cancer tissues, but does not react with normal cells or tissues. That is, since the antibody or fragment thereof of the present disclosure specifically reacts with cancer cells or cancer tissues, the pharmaceutical composition of the present disclosure can be suitably used for diagnosis of cancer. In the present specification, the pharmaceutical composition for diagnosis of cancer may be referred to as "the pharmaceutical composition for diagnosis of cancer of the present disclosure."

When the pharmaceutical composition of the present disclosure is used for diagnosis of cancer, it is preferred that the antibody or fragment thereof is labeled with a labeling substance, such as an enzyme (e.g., horseradish peroxidase (HRP), alkaline phosphatase (ALP), or β-D-galactosidase) or a fluorescent dye.

The pharmaceutical composition for diagnosis of cancer of the present disclosure can be used for diagnosing the presence or absence of cancer and the degree of cancer by reacting with a biological sample, and checking the presence or absence and degree of immune reaction.

Examples of cancers targeted by the pharmaceutical composition for diagnosis of cancer of the present disclosure include colorectal cancer such as colon cancer and rectal cancer; oral cancer such as squamous cell carcinoma; and the like. Thus, the pharmaceutical composition for diagnosis of cancer of the present disclosure is suitable as a composition for diagnosis of colorectal cancer. The pharmaceutical composition for diagnosis of cancer of the present disclosure is also suitable as a composition for diagnosis of oral cancer.

Examples of biological samples include body fluids, tissues, cells, and the like collected from a subject. Examples of body fluids include urine, serum, plasma, blood (whole blood), large-intestinal fluid, saliva, cerebrospinal fluid, synovial fluid, lymph fluid, amniotic fluid, ascitic fluid, pleural effusion, milk, bile, various tissue fluids, and the like. Examples of tissues include large-intestinal tissues, such as colon tissues and rectal tissues; oral tissues; and the like. Examples of cells include epithelial cells, mucosal cells, and the like.

The subject from which the biological sample is collected is preferably a mammal. Not only humans, but also non-human mammals can be used as subjects. Examples of human subjects include cancer patients, humans suspected of having cancer, and the like. Specific examples of cancers include colorectal cancer such as colon cancer and rectal cancer; oral cancer such as squamous cell carcinoma; and the like. Examples of non-human mammals include mammals kept as pet animals, livestock, laboratory animals, etc. Specific examples of such non-human mammals include dogs, cats, monkeys, bovine, horses, sheep, goats, pigs, rabbits, mice, rats, camels, llamas, and the like.

The method of collecting a biological sample is not particularly limited and can be any method known in this technical field.

The method of reacting the pharmaceutical composition for diagnosis of cancer of the present disclosure with a biological sample is not particularly limited and can be any method known in this technical field.

The presence or absence and degree of immune reaction can be checked, for example, by detecting a signal from a labeling substance described above. The method of detecting a signal is not particularly limited and can be any method known in this technical field.

As shown in the Examples described later, it has been confirmed that the overall survival rate and progression-free survival (PFS) of humans diagnosed with colorectal cancer worsen when the immunoreactivity to the antibody or fragment thereof of the present disclosure is high. Thus, the prognosis of colorectal cancer can be diagnosed (predicted) by comparing the immunoreactivity to the antibody or fragment thereof of the present disclosure with a set reference value. That is, the pharmaceutical composition for diagnosis of cancer of the present disclosure (more specifically, the pharmaceutical composition for diagnosis of colorectal cancer) can be more suitably used for diagnosis (prediction) of prognosis of colorectal cancer.

The immunoreactivity can be evaluated, for example, by the proportion of cells in which a signal from a labeling substance is detected (in other words, the proportion of cells expressing annexin A2 having an N-linked glycan relative to the total number of cells).

For example, a proportion of 50% or more can be set as a reference value. The immunoreactivity in a biological sample collected from the same subject in the past can also be used as a reference value.

When the immunoreactivity in a biological sample is higher than the reference value, it is predicted that there is a possibility that the prognosis of colorectal cancer in a subject from whom the biological sample was collected will worsen. On the other hand, when the immunoreactivity in a biological sample is lower than the reference value, it is predicted that there is a possibility that the prognosis of colorectal cancer in a subject from whom the biological sample was collected will be good.

As shown in the Examples described later, the antibody or fragment thereof of the present disclosure reacts not only with oral squamous cell carcinoma, but also with dysplastic epithelium. Thus, the pharmaceutical composition for diagnosis of cancer (more specifically, the pharmaceutical composition for diagnosis of oral cancer) of the present disclosure can be more suitably used for diagnosis of a precancerous lesion of oral cancer. Examples of precancerous lesions include epithelial dysplasia and the like.

As shown in the Examples described later, the antibody or fragment thereof of the present disclosure reacts with cancer cells or cancer tissues, but does not react with normal cells or tissues. Thus, since the antibody or fragment thereof of the present disclosure specifically reacts with cancer cells or cancer tissues, the pharmaceutical composition of the present disclosure can be suitably used for the treatment of cancer. In the present specification, the pharmaceutical composition for the treatment of cancer may be referred to as "the pharmaceutical composition for the treatment of cancer of the present disclosure."

For example, as shown in the Examples described later, the antibody or fragment thereof of the present disclosure has a tumor volume suppression effect. Thus, the pharmaceutical composition of the present disclosure, which comprises the antibody or fragment thereof of the present disclosure, can be suitably used for the treatment of cancer.

In addition, for example, the pharmaceutical composition for the treatment of cancer of the present disclosure may further comprise a compound having a therapeutic effect on cancer. The compound may be attached to the antibody or fragment thereof. In other words, for example, an antibody-drug conjugate (ADC) or the like may be used.

Examples of cancers targeted by the pharmaceutical composition for the treatment of cancer of the present disclosure include colorectal cancer such as colon cancer and rectal cancer; oral cancer such as squamous cell carcinoma; and the like. Thus, the pharmaceutical composition for the treatment of cancer of the present disclosure is suitable as a composition for the treatment of colorectal cancer. The pharmaceutical composition for the treatment of cancer of the present disclosure is also suitable as a composition for the treatment of oral cancer.

The subject that takes the pharmaceutical composition for the treatment of cancer of the present disclosure is preferably a mammal. Not only humans, but also non-human mammals can be used as subjects. Examples of human subjects include cancer patients, humans suspected of having cancer, and the like. Specific examples of cancers include colorectal cancer such as colon cancer and rectal cancer; oral cancer such as squamous cell carcinoma; and the like. Examples of non-human mammals include mammals kept as pet animals, livestock, laboratory animals, etc. Specific examples of such non-human mammals include dogs, cats, monkeys, bovine, horses, sheep, goats, pigs, rabbits, mice, rats, camels, llamas, and the like.

Examples of administration methods for the pharmaceutical composition for the treatment of cancer of the present disclosure include oral administration, parenteral (e.g., intravenous, intraarterial, intramuscular, subcutaneous, intraperitoneal, rectal, transdermal, and local) administration, and the like.

The dose (intake) of the pharmaceutical composition for the treatment of cancer of the present disclosure is not particularly limited and is determined according to the age, body weight, sex, and severity of symptoms of a subject to which the pharmaceutical composition is administered, the administration method, etc.

The present disclosure also includes use of annexin A2 having an N-linked glycan as a colorectal cancer marker.

The present disclosure also includes use of annexin A2 having an N-linked glycan as an oral cancer marker.

As described above, since the antibody or fragment thereof of the present disclosure recognizes the N-linked glycan that annexin A2 has, cancer can be diagnosed by evaluating the immunoreactivity. Thus, annexin A2 having an N-linked glycan is suitably used as a cancer marker.

Use of the cancer marker, more specifically, a colorectal cancer marker, enables diagnosis of colorectal cancer, diagnosis of prognosis of colorectal cancer, and the like, as described above.

Use of the cancer marker, more specifically, an oral cancer marker, enables diagnosis of oral cancer, diagnosis of a precancerous lesion of oral cancer, which is difficult to confirm even by histopathological diagnosis at present, and the like, as described above.

In the present specification, the term "comprising" includes "consisting essentially of" and "consisting of." Further, the present disclosure includes any and all combinations of the elements described herein.

Various characteristics (properties, structures, functions, etc.) described in the above embodiments of the present disclosure may be combined in any manner to specify the subject matter included in the present disclosure. That is, this disclosure includes all of the subject matter comprising any combination of the combinable properties described herein.

Examples

The contents of the present disclosure are described in detail with reference to the following Experimental Examples. However, the present disclosure is not limited thereto. In the following, experiments were performed at atmospheric pressure and ordinary temperature, unless otherwise specified. The term "%" indicates "mass%," unless otherwise specified. The amount of each component shown in each table is also shown as "mass%," unless otherwise specified.

Cell Culture

Human colon adenocarcinoma-derived HT-29 cells were cultured with 1 mM sodium butyrate (Tokyo Chemical Industry Co., Ltd., Tokyo, Japan) for 48 hours to induce differentiation. As an index of cell differentiation, alkaline phosphatase activity was measured using p-nitrophenyl phosphate (Sigma-Aldrich, St. Louis, MO, USA) as a substrate at 37°C for 15 minutes. The reaction was stopped by the addition of NaOH. The amount of p-nitrophenol was determined by measuring absorbance at 405 nm.

Generation of Monoclonal Antibody

Five BALB/c mice were immunized intraperitoneally every week with $1 \times 10^7$ HT-29 cells induced with sodium butyrate. Serum was obtained from the mice after the third immunization. IgG was purified from the serum using a HiTrap Protein G HP column (GE Healthcare, Germany) and bound to M-270 epoxy magnetic beads according to the manufacturer's protocol. Cell lysate proteins were isolated from HT-29 cells (without sodium butyrate treatment) using a cell lysis buffer. The solubilized protein mixture was adsorbed to the M-270 epoxy magnetic beads containing an antibody against the sodium butyrate-induced HT-29 cells, and then used for immunization to generate a monoclonal antibody.

A monoclonal antibody was generated according to the hybridoma technology of Kohler and Milstein. Hybridoma clones were screened using the following two-step process and isolated by limiting dilution.

Clones producing an antibody that reacted with HT-29 cells but did not react with sodium butyrate-treated differentiated HT-29 cells were first selected by immunofluorescence staining.

Subsequently, whether formalin-fixed, paraffin-embedded colorectal cancer tissue sections and oral cancer can be immunostained with candidate antibodies was investigated. The subclass of the antibodies was determined using IsoQuick (EnviroLogix, Inc., Portland, ME, USA). The antibodies were purified from culture supernatants using ImmunoAssist MG-PP (Kanto Chemical Co., Inc., Tokyo, Japan).

Immunofluorescence Staining

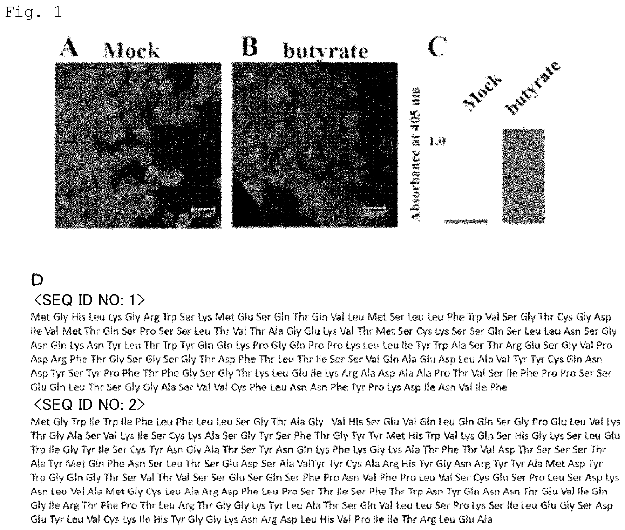

Cells were fixed with 4% (m/v) paraformaldehyde, permeabilized with 0.1% TritonX-100, and blocked with 10% goat serum. Thereafter, the cells were incubated using a culture supernatant containing 12G5A and then cultured using an Alexa Fluor 488-conjugated anti-mouse antibody. Images were acquired using a confocal laser scanning microscope (Leica TCS SP8, Germany). The 12G5A immunoreactivity was visualized with green fluorescence, and the nuclei were stained with DAPI. Fig. 1 shows the results of immunofluorescence staining of the obtained monoclonal antibody (12G5A).

It was confirmed that HT-29 colon cancer cells (Fig. 1A) were more reactive with antibody 12G5A than sodium butyrate-induced HT-29 cells (Fig. 1B). This suggests that antibody 12G5A is cancer cell-specific.

As shown in Fig. 1C, the alkaline phosphatase activity was enhanced when sodium butyrate was added, confirming that cells used in Fig. 1B had undergone differentiation.

Regarding the monoclonal antibody (12G5A), the amino acid sequence (SEQ ID NO: 1) of the light-chain variable region and the amino acid sequence (SEQ ID NO: 2) of the heavy-chain variable region were analyzed by sequence analysis (Fig. 1D).

Peptide Mass Fingerprinting and Matrix-Assisted Laser Desorption/Ionization Time-of-Flight (MALDI-TOF) Mass Spectrometry

Purified antibody 12G5A was bound to M-270 epoxy magnetic beads according to the manufacturer's protocol. 12G5A antigen was obtained from a cell lysate of SW480 cells by affinity chromatography using the M-270 epoxy magnetic beads to which antibody 12G5A was bound. The approximately 40 kDa protein band was stained with a Coomassie Brilliant Blue staining kit (Integrale, Tokushima, Japan), excised from the SDS-PAGE gel, digested with trypsin, and analyzed with MALDI-TOF MASS SPEC ANALYSIS (Genomine, Seoul, Korea).

The results of MALDI-TOF mass spectrometry suggest that the approximately 40 kDa protein is human annexin A2.

siRNA-mediated Gene Silencing

For silencing of annexin A2, Thermo Fisher Scientific siRNA (Waltham, MA, USA), Cat No. 4390824: s1383 and s9548 was used. Trilencer-27 Universal scrambled negative control siRNA (OriGene Technologies, Rockville, MD, USA) was used as a non-silencing control. Cells were transfected with the siRNAs using Lipofectamine(trademark) RNAiMAX (Invitrogen, Carlsbad, CA, USA) according to the manufacturer's instructions. The cells were used for subsequent studies 48 hours after transfection.

Immunoblotting

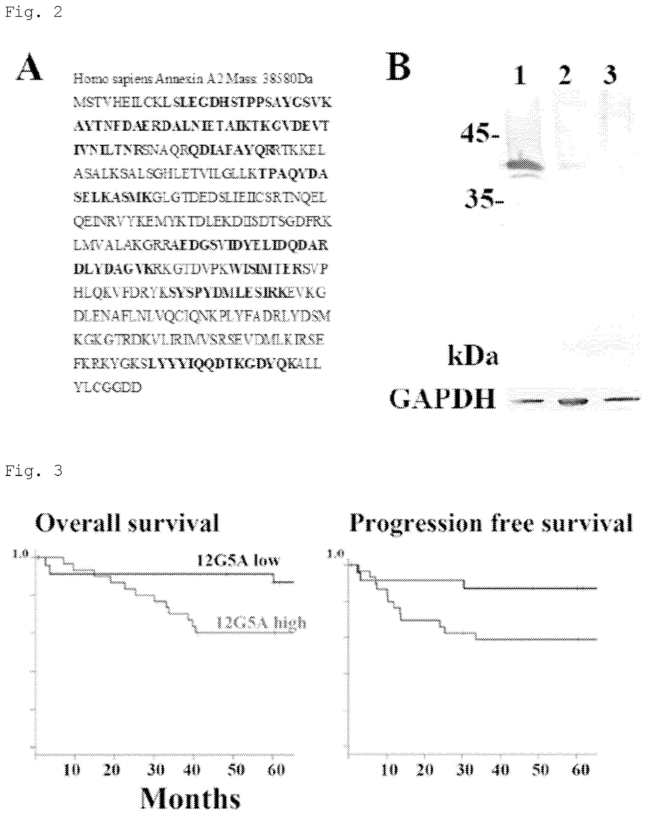

Proteins were electrophoresed on a sodium dodecyl sulfate-polyacrylamide gel electrophoresis (SDS-PAGE) gel and electroblotted onto a polyvinylidene difluoride membrane (Millipore, Bedford, MA, USA). The membrane was blocked with Block Ace (blocking milk; Yukijirushi, Sapporo, Japan) and then incubated with 0.5 ug/ml of 12G5A or anti-GAPDH antibody (Sigma-Aldrich, St. Louis, MO, USA). The immunoreactivity was evaluated using a Western blotting detection kit (Promega, Madison, WI, USA). Fig. 2B shows the results of Western blotting (lane 1: Mock siRNA; lane 2: s1383 siRNA; lane 3: s9548 siRNA).

The results of siRNA targeting and immunoblotting confirmed that 12G5A reacted with annexin A2.

Patients and Histopathological Classification

After receiving approval from the Institutional Review Board of the Gifu University Graduate School of Medicine (specific approval numbers: 2019-202, 2019-0444, 28-421, 2019-157, and 28-524) to carry out study, 55 specimens were collected from surgically treated patients who were primarily diagnosed with colorectal cancer.

All of the 55 primary tumors were examined macroscopically and microscopically to determine the depth of invasion, lymph node status, and distant metastasis to TNM classification. Invasion into lymphatic and blood vessels was determined according to Japanese classification of colorectal cancer 8th edition. In this classification, the status of cancer invasion to lymphatic (ly) and blood vessels (v) was histopathologically determined as ly0 and v0 (no invasion); ly1 and v1 (mild invasion); ly2 and v2 (moderate invasion); and ly3 and v3 (severe invasion). The infiltrative pattern of cancer was histopathologically classified as INFa (expansive growth), INFb (intermediate type), and INFc (infiltrative growth).

Immunohistochemical Staining

A rabbit monoclonal antibody against annexin A2 was purchased from CST (D11G2, Cambridge, MA, USA). All tissue specimens were fixed with 10% buffered formalin and embedded in paraffin. The tissues were immunostained as described above. Briefly, deparaffinized tissue slices were incubated in 10% normal goat serum for 30 minutes. Subsequently, the slides were incubated with 2 ug/ml of antibody 12G5A or D11G2 for 1 hour at room temperature or overnight at 4°C. For 12G5A, the tissue specimens were incubated with a goat anti-murine immunoglobulin $\mu$ chain antibody conjugated with horseradish peroxidase (1:200) (Abcam, cat# ab98679; Cambridge, MA, USA). For D11G2, the tissues were immunostained with an antibody attached to ImmPRESS (trademark) polymerized reporter enzyme staining system (Vector Laboratories, Burlingame, CA, USA). Finally, the reaction was developed with 3,3'-diaminobenzidine, and counter staining was performed with hematoxylin.

Evaluation of Immunohistochemical Staining and Statistical Analysis

The results obtained from immunohistochemical staining were expressed as a percentage calculated as the proportion of immunoreactive colorectal cancer cells relative to the total number of cells. The proportion of 12G5A immunoreactivity positive cells was determined by scoring 10 high-power fields for each sample. In staining, the immunoreactivity was considered "low" if less than 50% of the cancer cells exhibited immunoreactivity and "high" if 50% or more of the cancer cells exhibited immunoreactivity.

Table 1 shows the relationship between 12G5A immunoreactivity and clinical pathological features in 24 cases with low 12G5A immunoreactivity and 31 cases with high 12G5A immunoreactivity.

**Table 1: Correlation between 12G5A immunoreactivity and pathological features.**

| Factor | low 12G5A low(n=24) | high 12G5A (n=31) | P value |
|---|---|---|---|
| age(median) | 63.5 | 63.0 | 0.993 |
| male (%) | 12 (50.0) | 17 (54.8) | 0.789 |
| Distant.metastasis (%) | 3 (12.5) | 10 (32.3) | 0.116 |
| INFc (%) | 1 (42) | 4 (12.9) | 0.373 |
| Lymph node metastasis (%) | 15 (62.5) | 19 (61.3) | 1.000 |
| ly2 or ly3 (%) | 10 (41.7) | 13 (48.4) | 0.785 |
| Right colon (%) | 5 (20.8) | 8 (25.8) | 0.756 |
| pT3 or pT4 (%) | 23 (95.8) | 26 (83.9) | 0.216 |
| v2 or v3 (%) | 10 (41.7) | 14 (45.2) | 1.000 |

Overall survival (OS) and progression-free survival (PFS) curves were drawn using the Kaplan-Meier method, and differences in survival rates were compared using the log-rank test for univariate survival analysis. Multivariate Cox proportional hazards regression analysis was also performed to calculate the hazard ratio of death for 12G5A epitope expression. Fig. 3 and Table 2 show the results.

**Table 2**

**Univariate and multivariate analyses of risk factors for overall survival**

| | | Univariate analysis | | | Multivariate analysis | | |
| --- | --- | --- | --- | --- | --- | --- | --- |
| | n | Hazard Ratio | 95%CI | p | Hazard Ratio | 95%CI | p |
| Age (65-) | 26 | 1.01 | 0.36-2.78 | 0.99 | | | |
| Sex (male) | 29 | 1.12 | 0.43-3.37 | 0.73 | | | |
| 12G5A | 31 | 3.49 | 0.98-12.4 | 0.04 | 1.43 | 0.33-6.15 | 0.63 |
| pT3 or pT4 | 49 | | 0-Inf | 0.13 | | | |
| INFc | 5 | 5.76 | 1.79-18.5 | 0.67 | 1.60 | 0.44-5.87 | 0.48 |
| Lymph node metastasis | 34 | 3.04 | 0.86-10.8 | 0.07 | | | |
| ly2 or ly3 | 25 | 4.67 | 1.48-14.7 | 0.004 | 2.13 | 0.58-7.83 | 0.25 |
| v2 or v3 | 24 | 1.24 | 0.45-3.43 | 0.67 | | | |
| Distant metastasis | 13 | 10.8 | 3.62-32.0 | 1.37e-07 | 6.43 | 1.73-24.0 | 0.006 |

Log-rank *p*-value

As shown in Fig. 3, the overall survival rate and progression-free survival (PFS) of patients with high 12G5A immunoreactivity were significantly worse than those of patients with low 12G5A immunoreactivity (overall survival rate: P = 0.039, PFS: P = 0.033).

In addition, as shown in Table 2, multivariate analysis of survival risk showed that 12G5A immunoreactivity was not independently associated with prognosis (it was correlated with the T factor of colorectal cancer and thus not an independent prognostic factor).

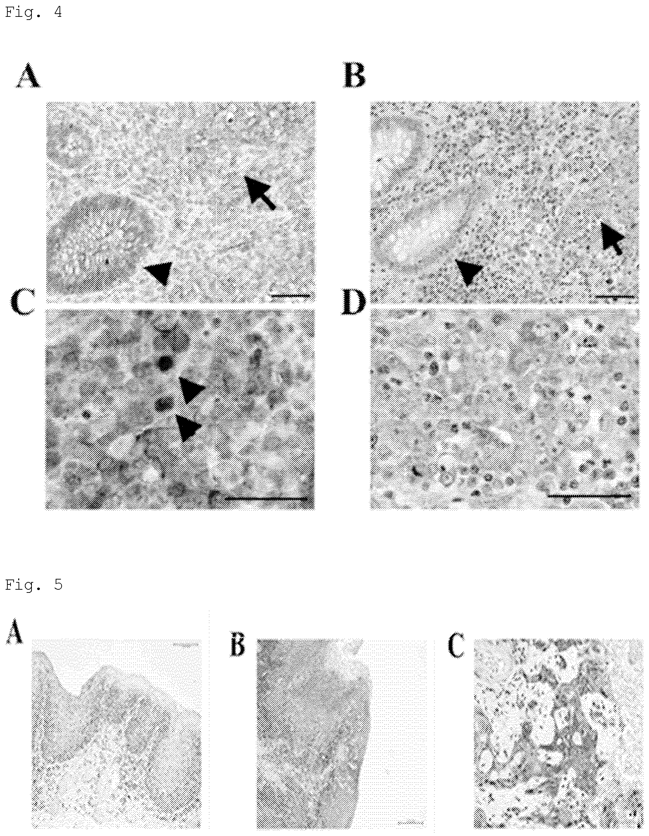

Fig. 4 shows the results of immunohistochemical staining using antibody 12G5A or D11G2. Scale bar represents 50 μm.

Fig. 4A shows the results of staining with D11G2, and Fig. 4B shows the results of staining with 12G5A. A commercially available specific antibody against annexin A2 (D11G2) was found to immunostain both noncancerous gland cells (indicated by arrowhead) and invasive colorectal adenocarcinoma cells (indicated by arrow) (Fig. 4A).

The specimen used in Fig. 4A was stained with 12G5A, and the tissue specimen was considered to have low 12G5A immunoreactivity (Fig. 4B). This suggests that immunoreactivity with antibody 12G5A is not necessarily the same as the immunoreactivity with commercially available antibody D11G2.

Fig. 4C shows a case of higher immunoreactivity with 12G5A than with D11G2. Strong annexin A2 immunoreactivity was observed at the cell surface, cytoplasm, and nucleus. 12G5A immunoreactivity is almost exclusively found in the cytoplasm. Fig. 4D shows a case of low immunoreactivity with 12G5A.

Fig. 5 shows the results of comparing 12G5A immunoreactivity in oral non-neoplastic stratified squamous epithelium (A), dysplastic epithelium (B), and oral squamous cell carcinoma (C) by immunohistochemical staining.

No staining was observed in oral non-neoplastic stratified squamous epithelium (A), whereas staining was observed in dysplastic epithelium (B) or oral squamous cell carcinoma (C).

Dysplastic epithelium is a precancerous state, and 12G5A was found to be immunoreactive not only to cancer cells but also to cells in a precancerous state.

Glycosidase Treatment

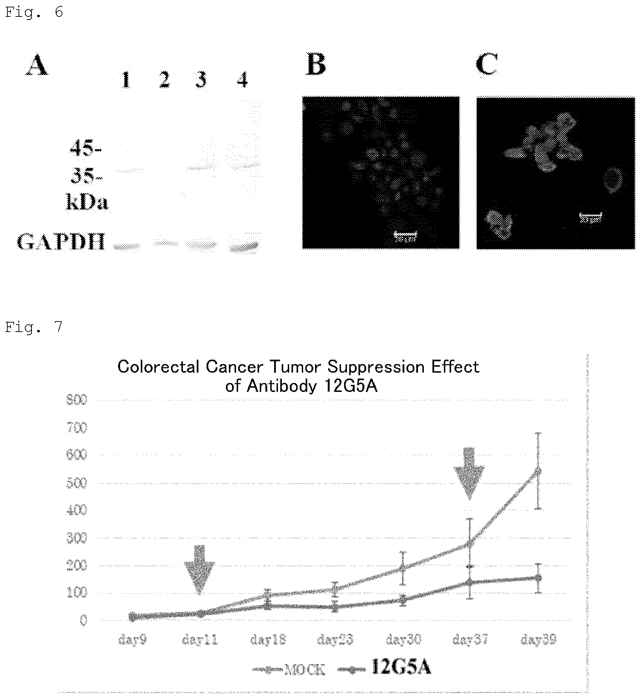

Glycopeptidase F was purchased from Takara Bio, Inc. O-glycosidase and $\alpha$2-3,6,8 neuraminidase were obtained from New England Biolabs (Beverly, MA, USA). Enzymatic deglycosylation of cell lysate protein was performed according to the manufacturer's protocol. Fig. 6A show Western blotting results (lane 1: no glycopeptidase F treatment (12G5A), lane 2: O-glycosidase and $\alpha$2-3,6,8 neuraminidase treatment (12G5A), lane 3: glycopeptidase F treatment (12G5A), lane 4: glycopeptidase F treatment (D11G2)).

Glycopeptidase F specifically cleaves the binding site (GlcNAc-Asn bond) between an N-glycan and a protein. However, glycopeptidase F does not act on glycans in which Fuc$\alpha$1-3 is attached to GlcNAc that is attached to Asn.

O-glycosidase cleaves Core 1 and Core 3 O-linked disaccharides from glycoproteins.

$\alpha$2-3,6,8 neuraminidase cleaves $\alpha$2-3, $\alpha$2-6, and $\alpha$2-8 linked N-acetylneuraminic acid residues from glycoproteins and oligosaccharides with high specificity. The cleavage efficiency of $\alpha$2-8 linkages is lower than that of $\alpha$2-3 and $\alpha$2-6 linkages.

As shown in Fig. 6A, it was confirmed that glycopeptidase F treatment reduced 12G5A immunoreactivity. On the other hand, the O-glycosidase or $\alpha$2-3,6,8 neuraminidase treatment did not reduce the immunoreactivity. The D11G2 immunoreactivity was not reduced by glycopeptidase F treatment.

SW480 cells were also incubated with or without 5 ug/ml of tunicamycin (Fujifilm Wako Pure Chemical Corporation, Osaka, Japan) for 48 hours to evaluate the effect of inhibition of N-linked glycosylation on immunofluorescence staining. Figs. 6B and 6C show the results. Blue indicates nuclear staining by DAPI.

Inhibition of N-linked glycosylation by tunicamycin abolished 12G5A immunoreactivity, shown in green, in SW480 cells (Fig. 6B) compared with SW480 cells without tunicamycin treatment (Fig. 6C).

These results suggest that 12G5A recognizes an N-linked glycosylation-modified tumor-associated epitope of annexin A2.

Colorectal Cancer Tumor Suppression Effect

On Day 0, $0.93 \times 10^7$ SW480 colorectal cancer cells were subcutaneously implanted in BALB/c nu/nu mice, and on Day 11 and Day 37, an antibody was administered (12G5A 0.9 mg/mouse). The tumor volume was measured over time (6 mice). The tumor volume was also measured in a group to which the antibody was not administered (MOCK, 5 mice).

$$\text{Tumor volume } (mm^2) = (\text{major axis of tumor; mm}) \times (\text{minor axis of tumor; mm})^2 \times 0.5236.$$

Fig. 7 shows the results.

As shown in Fig. 7, suppression of tumor volume was confirmed in the 12G5A administration group. In particular, on Day 39, tumor volume suppression was confirmed in the 12G5A administration group with significant difference (P <0.05, Student's t-test).

**Claims**

1. An antibody or fragment thereof that recognizes an N-linked glycan that annexin A2 has.

2. The antibody or fragment thereof according to claim 1, wherein the antibody or fragment thereof is an antibody or fragment thereof of (I) or (II):

   (I) an antibody or fragment thereof comprising a light-chain variable region consisting of the amino acid sequence set forth in SEQ ID NO: 1 and a heavy-chain variable region consisting of the amino acid sequence set forth in SEQ ID NO: 2; or
   (II) an antibody or fragment thereof comprising a light-chain variable region consisting of an amino acid sequence having at least 90% identity to the amino acid sequence set forth in SEQ ID NO: 1 and a heavy-chain variable region consisting of an amino acid sequence having at least 90% identity to the amino acid sequence set forth in SEQ ID NO: 2.

3. The antibody or fragment thereof according to claim 1 or 2, wherein the annexin A2 is a protein comprising the amino acid sequence set forth in SEQ ID NO: 3.

4. A pharmaceutical composition comprising the antibody or fragment thereof according to any one of claims 1 to 3.

5. The pharmaceutical composition according to claim 4, which is for use in diagnosis of cancer or for use in treatment of cancer.

6. The pharmaceutical composition according to claim 5, wherein the cancer is colorectal cancer or oral cancer.

7. Use of annexin A2 having an N-linked glycan as a colorectal cancer or oral cancer marker.

8. The use according to claim 7, wherein the annexin A2 is a protein comprising the amino acid sequence set forth in SEQ ID NO: 3.

Fig. 1

**A Mock** **B butyrate** **C**

D

**<SEQ ID NO: 1>**

Met Gly His Leu Lys Gly Arg Trp Ser Lys Met Glu Ser Gln Thr Gln Val Leu Met Ser Leu Leu Phe Trp Val Ser Gly Thr Cys Gly Asp
Ile Val Met Thr Gln Ser Pro Ser Ser Leu Thr Val Thr Ala Gly Glu Lys Val Thr Met Ser Cys Lys Ser Ser Gln Ser Leu Leu Asn Ser Gly
Asn Gln Lys Asn Tyr Leu Thr Trp Tyr Gln Gln Lys Pro Gly Gln Pro Pro Lys Leu Leu Ile Tyr Trp Ala Ser Thr Arg Glu Ser Gly Val Pro
Asp Arg Phe Thr Gly Ser Gly Ser Gly Thr Asp Phe Thr Leu Thr Ile Ser Ser Val Gln Ala Glu Asp Leu Ala Val Tyr Tyr Cys Gln Asn
Asp Tyr Ser Tyr Pro Phe Thr Phe Gly Ser Gly Thr Lys Leu Glu Ile Lys Arg Ala Asp Ala Ala Pro Thr Val Ser Ile Phe Pro Pro Ser Ser
Glu Gln Leu Thr Ser Gly Gly Ala Ser Val Val Cys Phe Leu Asn Asn Phe Tyr Pro Lys Asp Ile Asn Val Ile Phe

**<SEQ ID NO: 2>**

Met Gly Trp Ile Trp Ile Phe Leu Phe Leu Leu Ser Gly Thr Ala Gly  Val His Ser Glu Val Gln Leu Gln Gln Ser Gly Pro Glu Leu Val Lys
Thr Gly Ala Ser Val Lys Ile Ser Cys Lys Ala Ser Gly Tyr Ser Phe Thr Gly Tyr Tyr Met His Trp Val Lys Gln Ser His Gly Lys Ser Leu Glu
Trp Ile Gly Tyr Ile Ser Cys Tyr Asn Gly Ala Thr Ser Tyr Asn Gln Lys Phe Lys Gly Lys Ala Thr Phe Thr Val Asp Thr Ser Ser Ser Thr
Ala Tyr Met Gln Phe Asn Ser Leu Thr Ser Glu Asp Ser Ala ValTyr Tyr Cys Ala Arg His Tyr Gly Asn Arg Tyr Tyr Ala Met Asp Tyr
Trp Gly Gln Gly Thr Ser Val Thr Val Ser Ser Glu Ser Gln Ser Phe Pro Asn Val Phe Pro Leu Val Ser Cys Glu Ser Pro Leu Ser Asp Lys
Asn Leu Val Ala Met Gly Cys Leu Ala Arg Asp Phe Leu Pro Ser Thr Ile Ser Phe Thr Trp Asn Tyr Gln Asn Asn Thr Glu Val Ile Gln
Gly Ile Arg Thr Phe Pro Thr Leu Arg Thr Gly Gly Lys Tyr Leu Ala Thr Ser Gln Val Leu Leu Ser Pro Lys Ser Ile Leu Glu Gly Ser Asp
Glu Tyr Leu Val Cys Lys Ile His Tyr Gly Gly Lys Asn Arg Asp Leu His Val Pro Ile Ile Thr Arg Leu Glu Ala

Fig. 2

**A**

Homo sapiens Annexin A2 Mass: 38580Da

MSTVHEILCKL **SLEGDH** STPPSAYGSVK
**AYTNFDAER** RDALN **IE** TAIKTKGVDEVT
**IVN** ILTNR SNAQR **QDIAFAYQR** RTKKEL
ASALKSALSGHLETVILGLLK **TPAQYDA**
**SELKASMK** GLGTDEDSLIEIICSRTNQEL
QEINRVYKEMYKTDLEKDIISDTSGDFRK
LMVALAKGRRA **EDGSVIDYELIDQDAR**
**DLYDAG** VKRK GTDVPKWISIM **TERS** VP
HLQKVFDRYKSY **SPYDMLESIKKE** VKG
DLENAFLNLVQCIQNKPLYFADRLYDSM
KGK GTRDKVLIRIMVSRSEVDMLKIRSE
FKRKYGKS **LYYYIQQDTKGDYQK** ALL
YLCGGDD

**B**

1    2    3

45-

35-

kDa

GAPDH

Fig. 3

**Overall survival**

12G5A low

12G5A high

10   20   30   40   50   60

**Months**

**Progression free survival**

10   20   30   40   50   60

Fig. 4

Fig. 5

Fig. 6

Fig. 7

## INTERNATIONAL SEARCH REPORT

| | |
|---|---|
| | International application No. |
| | **PCT/JP2022/013783** |

**A. CLASSIFICATION OF SUBJECT MATTER**

*C07K 16/30*(2006.01)i; *A61K 39/395*(2006.01)i; *A61P 1/00*(2006.01)i; *A61P 1/02*(2006.01)i; *A61P 35/00*(2006.01)i; *C12N 15/13*(2006.01)i; *G01N 33/531*(2006.01)i; *G01N 33/574*(2006.01)i

FI: C07K16/30 ZNA; A61P35/00; A61K39/395 D; A61K39/395 N; A61K39/395 E; A61K39/395 T; G01N33/531 A; G01N33/574 A; C12N15/13; A61P1/00; A61P1/02

According to International Patent Classification (IPC) or to both national classification and IPC

**B. FIELDS SEARCHED**

Minimum documentation searched (classification system followed by classification symbols)

C07K16/30; A61K39/395; A61P1/00; A61P1/02; A61P35/00; C12N15/13; G01N33/531; G01N33/574

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN); GenBank/EMBL/DDBJ/GeneSeq; UniProt/GeneSeq; PubMed

**C. DOCUMENTS CONSIDERED TO BE RELEVANT**

| Category* | Citation of document, with indication, where appropriate, of the relevant passages | Relevant to claim No. |
|---|---|---|
| X | US 2020/0399353 A1 (AGENCY FOR SCIENCE, TECHNOLOGY AND RESEARCH) 24 December 2020 (2020-12-24)<br>claim 63, paragraphs [0005], [0055], [0114], [0116], [0175], [0178], [0194], [0203]. fig. 1B, 10B, 21A | 1-8 |

☐ Further documents are listed in the continuation of Box C.    ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **02 May 2022** | **17 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
| --- |
| **PCT/JP2022/013783** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
Information on patent family members

International application No.

**PCT/JP2022/013783**

| Patent document cited in search report | Publication date (day/month/year) | Patent family member(s) | Publication date (day/month/year) |
|---|---|---|---|
| US 2020/0399353 A1 | 24 December 2020 | WO 2018/021972 A1<br>EP 3490601 A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015119180 A